(19) Europäisches Patentamt
European Patent Office
Office européen des brevets

(11) EP 3 363 781 A1

# (12) EUROPÄISCHE PATENTANMELDUNG

(43) Veröffentlichungstag:
**22.08.2018 Patentblatt 2018/34**

(51) Int Cl.:
***C07C 213/00*** $^{(2006.01)}$ ***C07C 215/42*** $^{(2006.01)}$

(21) Anmeldenummer: **18156018.6**

(22) Anmeldetag: **09.02.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(30) Priorität: **15.02.2017 DE 102017202404**

(71) Anmelder: **Evonik Degussa GmbH**
**45128 Essen (DE)**

(72) Erfinder:
- **Rüfer, Alexander Martin**
  **45657 Recklinghausen (DE)**
- **Rittsteiger, Anne**
  **4233 Meltingen (CH)**
- **Poss, René**
  **63450 Hanau (DE)**
- **Roos, Meike**
  **63654 Büdingen (DE)**
- **Röder, Stefan**
  **36391 Sinntal (DE)**
- **Berweiler, Monika**
  **63477 Maintal (DE)**

## (54) VERFAHREN ZUR HERSTELLUNG VON ISOPHORONAMINOALKOHOL (IPAA)

(57) Vorliegende Erfindung betrifft ein verbessertes Verfahren zur Herstellung von 3-(Aminomethyl)-3,5,5-trimethylcyclohexanol, im folgenden Isophoronaminoalkohol (IPAA) genannt.

EP 3 363 781 A1

**Beschreibung**

Vorliegende Erfindung betrifft ein verbessertes Verfahren zur Herstellung von 3-Aminomethyl-3,5,5-trimethylcyclohexanol aus Isophoronnitril, im folgenden Isophoronaminoalkohol (IPAA) genannt.

IPAA stellt ein wichtiges Zwischenprodukt in verschiedenen Anwendungsbereichen dar. So dient es beispielsweise als Vorstufe pharmakologischer Produkte, insbesondere im Bereich der Influenza-Prophylaxe (WO 2011/095576). Weitere Anwendungen beinhalten u.a. den Einsatz in Polymeren, Korrosionsschutzmitteln und Stabilisatoren (DE 1229078).

Die bisherigen Verfahren zeichnen sich dabei durch vergleichsweise komplizierte Reaktionsführung und niedrige Ausbeuten aus.

Pandey et al. (Indian Journal of Chemistry, Vol. 43B, 2004, 2705-2707) beschreibt eine Syntheseroute zu IPAA, ausgehend von Isophoronnitril (IPN). Dabei wird über mehrere Stufen vorgegangen: Zunächst erfolgt die Bildung des korrespondierenden Amids über Hydrolyse unter basischen Bedingungen. Anschließend erfolgen die Reduktion der Ketofunktion mittels Cyanoborhydrid und die Reduktion des Amids mittels Lithiumaluminiumhydrid.

Eine ähnliche Vorgehensweise ist in den Anmeldungen WO 2011/094953 und WO 2011/095576 beschrieben, hier erfolgt die Reduktion der Keto- und Nitrilgruppe von IPN jedoch ausschließlich mit Lithiumaluminiumhydrid in THF unter Rückfluss. Als nachteilig ist anzusehen, dass die o.g. Verfahren den stöchiometrischen Einsatz aufwendig herzustellender und damit teurer Reduktionsmittel vorsehen. Weiterhin erfolgt die Synthese über mehrere Stufen und ist damit aufwendig.

Die Anmeldung DE 1229078 beschreibt die Herstellung von IPAA aus IPN unter Zuhilfenahme von Ammoniak und Wasserstoff an einem Fischer-Tropsch-Katalysator und sehr hohem Wasserstoffpartialdruck. Die hierbei erforderlichen, relativ hohen Reaktionstemperaturen begünstigen jedoch im Falle von gamma-Ketonitrilen (wie IPN) die Abspaltung von Blausäure (HCN). Dieser Umstand führt zur Ausbeuteminderung und ist daher nachteilig. Als weiterer Nachteil ist anzusehen, dass HCN stark toxisch auf Organismen wirkt (Gefahr bei Freisetzung) und üblicherweise zur Katalysatordesaktivierung beiträgt. Ferner erfolgt substanzielle Dimerenbildung aufgrund der Reaktion der entstehenden Amingruppe mit noch nicht vollständig reduzierter Ketogruppe. Dies stellt ebenso einen Ausbeuteverlust bezogen auf das Wunschprodukt IPAA dar.

Es ist auch bekannt aus EP 42119, EP 659734, EP 503246, WO 2012/076315, dass sich bei der konventionellen Hydrierung von IPN, das IPAA nur als Nebenprodukt in unerwünschter Weise bildet, was auch als Nachteil bezeichnet wird. Auch hier wird auf die Abspaltung von Blausäure (HCN) hingewiesen. Diese Nachteile können durch den Einsatz von granulierten Raney-Typ-Katalysatoren aus EP 3050870 umgangen werden.

Reaktionsschema:

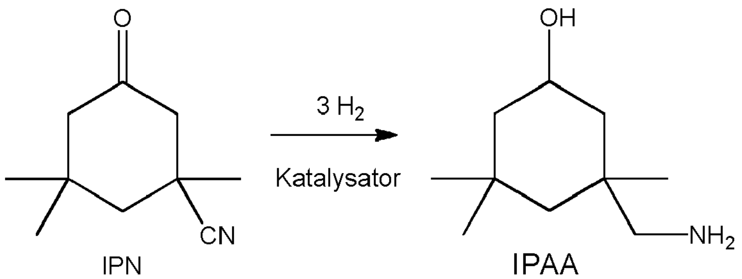


IPN IPAA

Die zugrundeliegende Aufgabe war es, ein Verfahren zur Herstellung von 3-Aminomethyl-3,5,5-trimethylcyclohexanol (IPAA), durch Hydrierung von Isophoronnitril (IPN) mit Hilfe von Wasserstoff an einem Katalysator zu finden, wobei der Katalysator eine zum einen möglichst geringe Schüttdichte aufweisen soll, und zum anderen eine hohe Aktivität für die genannte Hydrierreaktion aufweist.

Werden für die Herstellung von IPAA durch Hydrierung von IPN Raney-Cobalt-Katalysatoren vom Granulattyp gemäß EP 3050870 eingesetzt, so weisen diese üblicherweise eine Schüttdichte von mehr als 1,5 kg/L auf. Bei üblichen Reaktorgrößen von 5 bis 50 $m^3$, entspricht dies einer erforderlichen Cobalt-Menge pro Katalysatorfüllung von 8 bis 100 Tonnen.

Überraschend wurde nun gefunden, dass der erfindungsgemäße Katalysator, bestehend aus katalytisch aktiven Metallschaumkörpern, die Aufgabe der Erfindung löst.

Unter Metallschäumen werden feste metallische Schäume verstanden, die eine hohe Porosität und zahlreiche gegenseitige Verbindungen zwischen Bereichen festen Materials mit irregulärer Struktur aufweisen. Solche Metallschäume werden auch "zellulare Metalle" genannt, wobei der Begriff "Metallschäume" verbreiteter ist. Der Begriff "Metallschäume" ist in der Fachliteratur gut etabliert und zum Beispiel in Ullmann’s Encyclopedia of Industrial Chemistry, Kapitel "Metallic Foams", veröffentlicht online am 15.07.2012, DOI: 10.1002/14356007.c16_c01.pub2 näher erläutert.

Unter "katalytisch aktiven Metallschaumkörpern" im Sinne dieser Erfindung werden Metallschaumkörper verstanden, die keine zusätzliche Beschichtung aufweisen, welche gegebenenfalls eine Verengung der Poren in den Metallschaumkörpern bewirken können. Katalytisch aktive Metallschaumkörper im Sinne dieser Erfindung sind ohne zusätzliche, gegebenenfalls katalytisch aktive Beschichtungen in der Zielreaktion - hier: in der Umsetzung von IPN zu IPAA - katalytisch aktiv.

Gegenstand der Erfindung ist ein einstufiges Verfahren zur Herstellung von 3-Aminomethyl-3,5,5-trimethylcyclohexanol, durch Hydrierung von Isophoronnitril (IPN) mit Wasserstoff an einem Katalysator, in An- oder Abwesenheit von Lösungsmittel, wobei der Katalysator die folgenden Eigenschaften aufweist:

I. Der Katalysator liegt in Form von katalytisch aktiven Metallschaumkörpern vor und weist eine Schüttdichte von nicht mehr als 0,8 kg/L auf.

und

II. Der Katalysator weist im aktivierten Zustand in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 70 bis 99,8 Gew.-% |
| Aluminium: | 0,02 bis 15 Gew.-% |

sowie eines oder mehrere Elemente ausgewählt aus der Gruppe bestehend aus Chrom, Nickel und

| | |
|---|---|
| Eisen: | 0 bis 15 Gew.-%, |

und

III. die katalytisch aktiven Metallschaumkörper weisen eine durchschnittliche Teilchengröße von 1 bis maximal 70 mm auf,

und

IV. die katalytisch aktiven Metallschaumkörper zeigen eine poröse Schaumstruktur, wobei die makroskopischen Poren Größen im Bereich von 100 bis 5000 μm, bevorzugt von 200 bis 2500 μm, besonders bevorzugt von 400 bis 1200 μm aufweisen.

Katalysator:

Bestandteile

Der erfindungsgemäße Katalysator gehört zu den aktivierten Metallkatalysatoren. Aktivierte Metallkatalysatoren sind dem Fachmann auch als Katalysatoren vom "Raney-Typ", oder einfach "Raney-Katalysatoren" grundsätzlich bekannt. Sie werden im Allgemeinen hergestellt durch Bildung einer mindestens binären Metalllegierung aus mindestens einer gegebenenfalls katalytisch aktiven Metallkomponente und mindestens einer laugbaren Metallkomponente und anschließendes Herauslösen der laugbaren Metallkomponente.

Der Katalysator weist im aktivierten Zustand in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 70 bis 99,8 Gew.-% |
| Aluminium: | 0,02 bis 15 Gew.-% |

sowie eines oder mehrere Elemente, ausgewählt aus der Gruppe bestehend aus Chrom, Nickel und

| | |
|---|---|
| Eisen: | 0 bis 15 Gew.-%. |

Bevorzugte Ausführungsform

Der erfindungsgemäße Katalysator enthält 70 bis 99,8 Gew.-%, bevorzugt 70 bis 95 Gew.-%, besonders bevorzugt 80 bis 90 Gew.-% Cobalt und 0,02 bis 15 Gew.-%, bevorzugt 4 bis 13 Gew.-%, besonders bevorzugt 6 bis 12 Gew.-% Aluminium und weiterhin enthalten bevorzugte Ausführungsformen des erfindungsgemäßen Katalysators 0 bis 15 Gew.-%, bevorzugt 0,05 bis 8 Gew.-%, besonders bevorzugt 0,1 bis 3,5 Gew.-% und ganz besonders bevorzugt 0,2

bis 2,5 Gew.-% eines oder mehrere Elemente ausgewählt aus der Gruppe bestehend aus Chrom, Nickel und Eisen. Besonders bevorzugt sind Chrom und Eisen. Die genannten Bereiche können beliebig miteinander kombiniert werden.

Weiterhin enthalten bevorzugte Ausführungsformen des erfindungsgemäßen Katalysators 0 bis 10 Gew.-% Dotiermetalle und/oder Promotoren zur Erhöhung der Aktivität, Selektivität und/oder Standzeit.

Bevorzugt enthalten sind ein oder mehrere Dotiermetalle ausgewählt aus der Gruppe bestehend aus Platin (Pt), Palladium (Pd), Rhodium (Rh), Ruthenium (Ru), Osmium (Os), Iridium (Ir), Molybdän (Mo), Wolfram (Wo), Mangan (Mn), Rhenium (Re), Kupfer (Cu), Silber (Ag) und Gold (Au) in einem Konzentrationsbereich von 0,05 bis 10 Gew.-%, besonders bevorzugt 0,2 bis 3,5 Gew.-%.

Besonders bevorzugt weist der Katalysator im aktivierten Zustand in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 80 bis 90 Gew.-% |
| Aluminium: | 6 bis 12 Gew.-% |

sowie eines oder mehrere Elemente ausgewählt aus der Gruppe bestehend aus Chrom, Nickel und

| | |
|---|---|
| Eisen: | 0,05 bis 8 Gew.-%. |

Ganz besonders bevorzugt weist der Katalysator im aktivierten Zustand in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 80 bis 90 Gew.-% |
| Aluminium: | 6 bis 12 Gew.-% |

sowie eines oder mehrere Elemente ausgewählt aus der Gruppe bestehend aus Chrom, Nickel und

| | |
|---|---|
| Eisen: | 0,1 bis 3,5 Gew.-%. |

Der erfindungsgemäße Katalysator wird durch Aktivierung des mit Aluminium und gegebenenfalls mit weiteren Metallen modifizierten Cobalt-Metallschaums gewonnen. Hierzu wird mindestens ein Teil des Aluminiums chemisch aus dem Legierungsmaterial herausgelöst ("gelaugt"). Dazu werden wässrige basische Lösungen eingesetzt, bevorzugt Alkalihydroxidlösungen.

Der erfindungsgemäße Katalysator wird aus einer mindestens binären Metalllegierung umfassend Aluminium (Al) und Cobalt (Co) und gegebenenfalls der weiteren Bestandteilen durch Herauslösen von Aluminium, hergestellt.

Eigenschaften

Schüttdichte

Der erfindungsgemäße Katalysator weist eine Schüttdichte von nicht mehr als 0,8 kg/l, bevorzugt von 0,1 bis 0,7 kg/l, besonders bevorzugt von 0,2 bis 0,6 kg/l auf.

Die Schüttdichte des erfindungsgemäßen Katalysators ist damit deutlich geringer als die aus dem Stand der Technik bekannter "Raney-Katalysatoren", die zur Herstellung von IPDA aus IPN zum Einsatz kommen.

Schüttdichte, auch Fülldichte ($d_{Sch}$) (Engl. bulk density, poured density) eines Feststoffes genannt, ist das Verhältnis der Masse zum Volumen eines Gemenges aus einem körnigen Feststoff und Luft, welche die Hohlräume zwischen den Partikeln ausfüllt. Dieser unter Fachleuten gängige Parameter wird mittels eines Messzylinders, durch Bestimmung der Masse ($M_F$) eines definierten Schüttungsvolumens an Feststoff ($V_F$) bestimmt:

$$d_{Sch} = M_F/V_F$$

Teilchengrößen

Der erfindungsgemäße Katalysator besteht aus katalytisch aktiven Metallschaumkörpern. Diese katalytisch aktiven Metallschaumkörper weisen bevorzugt eine durchschnittliche Teilchengröße von 1 bis maximal 70 mm auf. Grundsätzlich ist die optimale Teilchengröße steuerbar und wird auf die im Einsatzreaktor herrschenden Gegebenheiten abgestimmt. Bevorzugt wird der erfindungsgemäße Katalysator als Schüttgut aus katalytisch aktiven Metallschaumkörpern eingesetzt, wobei die katalytisch aktiven Metallschaumkörper eine Teilchengröße von 1 bis 50 mm, besonders bevorzugt von 1 bis 30 mm und ganz besonders bevorzugt von 1 bis 10 mm aufweisen. Die Teilchen einer Schüttung können dabei gleiche oder unterschiedliche Größen aufweisen.
Die durchschnittliche Teilchengröße für die Teilchen mit einer Größe im Bereich von 5 μm bis 125 mm kann durch Siebanalyse nach DIN 66165 bestimmt werden. Geeignete Methoden und Beschreibungen zur Siebanalyse sind beschrieben in:

DIN 66165-1:1987-04 Partikelgrößenanalyse; Siebanalyse; Grundlagen, und in DIN 66165-2:1987-04 Partikelgrößenanalyse; Siebanalyse; Durchführung.
Paul Schmidt, Rolf Körber, Matthias Coppers: Sieben und Siebmaschinen: Grundlagen und Anwendung. Wiley-VCH Verlag, 2003, ISBN 9783527302079, Kapitel 4.4: Analysesiebung.
Jörg Hoffmann: *Handbuch der Messtechnik.* Hanser Verlag, 2007, ISBN 978-3-446-40750-3, Kapitel 3.12.16.2.1.

Alternativ kann eine durchschnittliche Teilchengröße mittels eines Mikroskops optisch erfasst werden, wobei ein Zahlenmittelwert aus mindestens 100 einzelnen Werten zu bestimmen ist.

BET Oberfläche

Der erfindungsgemäße Katalysator weist eine BET Oberfläche von 1 bis 200 $m^2/g$, bevorzugt 10 bis 120 $m^2/g$, besonders bevorzugt 70 bis 100 $m^2/g$ auf. Die spezifische Oberfläche, auch vereinfacht BET Oberfläche genannt, wird nach DIN 9277 durch Stickstoffadsorption nach dem Brunauer-Emmett-Teller-Verfahren bestimmt, wie beschrieben in J. Am. Chem. Soc. 1938, Vol. 60, S. 309-319.

Porengrößen

Der erfindungsgemäße Katalysator besteht aus katalytisch aktiven Metallschaumkörpern. Metallschaumstrukturen, die viele Hohlräume enthalten, können zum Beispiel durch Einwirkung von Gasen auf ein verflüssigtes Metall und nachfolgende Abkühlung entstehen. Eine weitere Möglichkeit, um zu solchen Strukturen zu gelangen, besteht darin, organische Schaumstrukturen als Templat (Basis) für die Auftragung eines Metalls zu benutzen und anschließend das organische Templat durch Verbrennung zu entfernen.

Die katalytisch aktiven Metallschaumkörper, aus denen sich der erfindungsgemäße Katalysator zusammensetzt, zeigen bevorzugt eine poröse Schaumstruktur, wobei die makroskopischen Poren Größen im Bereich von 100 bis 5000 μm, bevorzugt von 200 bis 2500 μm, besonders bevorzugt von 400 bis 1200 μm aufweisen. Zur Bestimmung der Größe der makroskopischen Poren kann zum Beispiel eine in "The Guide 2000 of Technical Foams", Buch 4, Teil 4, Seiten 33-41 beschriebene Methode benutzt werden. Dabei kann die Größe der makroskopischen Poren durch eine optische Messung des Porendurchmessers einer ausgewählten Pore bestimmt werden. Diese Messung wird für mindestens 100 unterschiedliche Poren wiederholt und daraus ein Mittelwert des Porendurchmessers als Analyseergebnis berechnet.

Allgemeine Methode zur Herstellung des Katalysators

Zur Herstellung eines erfindungsgemäßen Katalysators wird ein kommerziell erhältlicher Cobalt-Metallschaum mit einem Haftvermittler besprüht, mit Aluminiumpulver und gegebenenfalls weiteren Metallpulvern wie beispielsweise Eisen und/oder Nickel und/oder Chrom beschichtet und das so erhaltene Material einer Wärmebehandlung unterzogen. Dann erfolgt eine Zerkleinerung, Vereinzelung und/oder Formgebung des nach der Wärmebehandlung erhaltenen Legierungsmaterials. Die katalytisch aktiven Metallschaumkörper, aus denen der erfindungsgemäße Katalysator besteht, werden aus den so erhaltenen Legierungsschaumkörpern durch Herauslösen mindestens eines Teils des darin enthaltenen Aluminiums hergestellt.

Der zur Herstellung des erfindungsgemäßen Katalysators zu verwendende Cobalt-Metallschaum wird bevorzugt in Plattenform mit Kantenlängen bis zu 500 mm und einer Dicke von nicht mehr als 5 mm eingesetzt. Um die Haftung von Aluminiumpulver und gegebenenfalls weiterer Metallpulver auf dem Cobalt-Metallschaum zu verbessern, wird dieser zunächst mit einem Haftvermittler behandelt. Jeder Haftvermittler, der die Haftung zwischen Metallen und organischen

Materialien verbessert, kann eingesetzt werden. Geeignet ist beispielsweise Polyethyleniminlösung.

Nach dem Auftragen des Aluminiumpulvers und gegebenenfalls weiterer Metallpulver auf den Cobalt-Metallschaum wird das Material einer Wärmebehandlung im Temperaturbereich von 500 bis 1000°C, bevorzugt von 600 bis 800°C unterzogen, wobei zunächst Feuchtigkeit und organische Reststoffe aus dem vorhergehenden Beschichtungsprozess entfernt und anschließend Aluminium mindestens teilweise verflüssigt und in die Cobalt-Metallschaumstruktur einlegiert wird. Die Wärmebehandlung erfolgt in einer Atmosphäre aus Sauerstoff-freiem Inertgas, um die Ausbildung störender oxidischer Schichten zu verhindern.

Das so erhaltene Material, ein mit Aluminium und gegebenenfalls mit weiteren Metallen modifizierter Cobalt-Metallschaum, wird dann gegebenenfalls zerkleinert, vereinzelt und/oder einer entsprechenden Formgebung unterzogen. Die Zerkleinerung und Vereinzelung des modifizierten Cobalt-Metallschaums kann beispielsweise durch Laserschneiden bzw. Laserstrahlschneiden erfolgen. Die entstehenden Materialstücke (Teilchen, Partikel) weisen bevorzugt eine Quader- oder Parallelepipedform mit einer maximalen Kantenlänge von nicht mehr als 50 mm auf und bilden in ihrer Gesamtheit ein Schüttgut.

Im nächsten Schritt wird der erfindungsgemäße Katalysator durch Aktivierung des mit Aluminium und gegebenenfalls mit weiteren Metallen modifizierten Cobalt-Metallschaums gewonnen. Hierzu wird mindestens ein Teil des Aluminiums chemisch aus dem Legierungsmaterial herausgelöst ("gelaugt"). Dazu werden wässrige basische Lösungen eingesetzt, bevorzugt Alkalihydroxidlösungen ausgewählt aus der Gruppe bestehend aus Natriumhydroxid-, Kaliumhydroxid- oder Lithiumhydroxidlösung, besonders bevorzugt ist wässrige Natriumhydroxidlösung. Die Konzentration der bei der Katalysatorherstellung eingesetzten wässrigen Alkalihydroxidlösung kann generell zwischen 0,1 und 60 Gew.-% Alkalihydroxid sein. Bevorzugt erfolgt das Herauslösen des Aluminiums (auch als Laugung bezeichnet) mit einer 5 bis 50 Gew.-%igen, besonders bevorzugt 5 bis 35 Gew.-%igen wässrigen Natriumhydroxidlösung bei einer Temperatur von 20 bis 100°C, bevorzugt bei 40 bis 85°C, besonders bevorzugt bei 50 bis 80°C. Die dabei anzuwendenden Laugzeiten, d.h. die Reaktionszeiten der Natriumhydroxidlösung mit dem mit Aluminium modifizierten Cobalt-Metallschaum, hängen außer von den sonstigen, vorstehend benannten Reaktionsbedingungen vom einzustellenden Aluminiumgehalt im Endprodukt ab und können zwischen 2 und 240 Minuten liegen.

Der durch Modifizierung und Aktivierung erzeugte erfindungsgemäße Katalysator behält die makroskopische Schaumstruktur des ursprünglich eingesetzten Cobalt-Metallschaums. Das mindestens teilweise Herauslösen des Aluminiums erfolgt in Oberflächen-nahen Bereichen, wo eine hochporöse, katalytisch aktive Cobalt-Struktur erzeugt wird. Die BET Oberfläche des erfindungsgemäßen Katalysators ist bevorzugt größer als die des eingesetzten Cobalt-Metallschaums.

Zur Verbesserung von Aktivität, Selektivität und/oder Standzeit des erfindungsgemäßen Katalysators können unterschiedliche Dotierungen und/oder Promotoren zugesetzt werden. Dies kann durch Einlegierung eines Dotierungsmetalls in den zur Katalysatorherstellung eingesetzten Cobalt-Metallschaum und/oder durch nasschemische Nachbehandlung des erfindungsgemäßen Katalysators erfolgen. Beispielsweise kann das Dotierungsmetall durch Fällung oder reduktive Zersetzung aus bevorzugt wässriger Lösung einer geeigneten Vorstufe aufgebracht werden. Bevorzugt werden ein oder mehrere Dotierungselemente aufgebracht, ausgewählt aus der Gruppe bestehend aus Platin (Pt), Palladium (Pd), Rhodium (Rh), Ruthenium (Ru), Osmium (Os), Iridium (Ir), Molybdän (Mo), Wolfram (Wo), Mangan (Mn), Rhenium (Re), Kupfer (Cu), Silber (Ag) und Gold (Au).

Der erfindungsgemäße Katalysator wird für die Herstellung von Isophoronaminoalkohol durch Hydrierung eingesetzt.

Die Hydrierung erfolgt einstufig in Batch-Autoklaven oder Festbettreaktoren. Geeignete Reaktortypen sind z.B. Rührkessel, Schachtöfen, Hordenreaktoren oder Rohrbündelreaktoren.

Der Prozess kann in diskontinuierlicher oder kontinuierlicher Fahrweise durchgeführt werden.

Die Hydrierung erfolgt üblicherweise bei Temperaturen zwischen 20 bis 200°C, bevorzugt 20 bis 150°C, besonders bevorzugt 40 bis 130°C, und Drücken von 0,3 bis 50 MPa, bevorzugt 0,3 bis 30 MPa, besonders bevorzugt 5 bis 15 MPa. Die Bereiche für Druck und Temperatur sind beliebig kombinierbar. Die Hydrierung kann in An- oder Abwesenheit von Lösungsmittel durchgeführt werden. Als Lösungsmittel eignen sich u.a. Ether, cyclische und lineare Kohlenwasserstoffe und Alkohole, bevorzugt werden Methanol, Ethanol oder THF verwendet.

Der für die Hydrierung erforderliche Wasserstoff kann dem Reaktor entweder im Überschuss, beispielsweise mit bis zu 10.000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

Das erforderliche Volumen der einzusetzenden Hydrierkatalysatoren richtet sich nach dem vom Betriebsdruck, der Temperatur, der Konzentration und der Katalysatoraktivität abhängigen LHSV-Wert (liquid hourly space velocity), der eingehalten werden muss, um eine möglichst vollständige Hydrierung des eingesetzten IPN zu gewährleisten. Üblicherweise liegt der LHSV-Wert bei der Verwendung des bevorzugt einzusetzenden Gemisches aus IPN und Wasserstoff zwischen 0,1 und 8 Liter Lösung pro Liter Katalysator und Stunde, bevorzugt zwischen 1 und 4 $l_{Lsg}\ l_{Kat}^{-1}\ h^{-1}$.

Bevorzugt wird die Hydrierung kontinuierlich in Festbettreaktoren durchgeführt, besonders bevorzugt kontinuierlich in Festbettreaktoren, die in Rieselbettfahrweise oder Sumpffahrweise betrieben werden.

Das die Hydrierung verlassende Reaktionsgemisch wird mit den üblichen Methoden weiter aufgereinigt, um ein IPAA mit der gewünschten Qualität zu erhalten. Dabei können alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden.

Bevorzugt wird die Aufreinigung durch Destillation unter Druck und/oder im Vakuum in mehreren Schritten erreicht. Hierfür lassen sich beliebige Destillationskolonnen mit und ohne Einbauten wie z.B. Dephlegmatoren, Trennwänden, ungeordnete Einbauten oder Schüttungen, geordnete Einbauten oder Packungen, Böden mit oder ohne Zwangsführung verwenden.

**Beispiele**

**Herstellung des Katalysators:**

Ein kommerziell als Rollenware erhältlicher Cobalt-Metallschaum mit einer Dicke von 1,9 mm, einer Breite von 300 mm und einer mittleren Porengröße von 580 μm wurde mit einer kommerziell erhältlichen Haftvermittlerlösung besprüht, mit Aluminiumpulver beschichtet und einer Wärmebehandlung bei 700°C unterzogen. Nach Abkühlung wurde das so erhaltene Material mit einem Laser in viereckige Teilchen mit einer Kantenlänge von 4 mm x 4 mm und einer Dicke von 1,9 mm geschnitten.

Das resultierende Schüttgut wurde zur katalytischen Aktivierung in einer Festbettschüttung angeordnet und durch Durchpumpen von 10 Gew.-%iger Natronlauge bei 60°C für die Dauer von 60 Minuten nasschemisch nachbehandelt. Das Material wurde anschließend mit Wasser solange gewaschen, bis ein pH-Wert der Waschlösung nach Durchpumpen durch die Festbettschüttung von kleiner 10 erreicht war.

Die Zusammensetzung der so erhaltenen, katalytisch aktiven Metallschaumkörper wurde per ICP-OES analysiert. Die Ergebnisse sind in der nachfolgenden Tabelle 1 angegeben:

Tabelle 1:

| Cobalt-Gehalt | Aluminium-Gehalt | Schüttdichte |
|---|---|---|
| 97,4 Gew.-% | 2,09 Gew.-% | 0,5 kg/L |

**Herstellung von IPAA mit dem erfindungsgemäßen Katalysator**

Der erfindungsgemäße Katalysator wurde bei der Herstellung von 3-Aminomethyl-3,5,5-Trimethylcyclohexanol (Isophoronaminoalkohol, IPAA) aus 3-Cyano-3,5,5-Trimethylcyclohexanon (Isophoronnitril, IPN) in einem diskontinuierlichen Verfahren in einem Laborautoklaven auf seine katalytische Wirksamkeit getestet.

Für die Durchführung der einzelnen Experimente wurde die Reaktionslösung aus 10 Gew.-% IPN und 90 Gew.-% THF im Reaktor vorgelegt. 150 ml des Katalysators befanden sich dabei in einem speziellen Katalysatorkorb. Nach dem Aufheizen auf die gewünschte Reaktionstemperatur wurde der gewünschte Reaktionsdruck durch Zugabe von Wasserstoff eingestellt. Die Untersuchung der Hydrierung von IPN mit den erfindungsgemäßen Katalysatoren erfolgte bei einer Temperatur von 100°C bei einem Druck von 50 bar.

Die Produktlösung wurde mittels Gaschromatographie auf IPN, IPAA und entsprechende Nebenkomponenten untersucht. Die Ergebnisse sind in Tabelle 2 aufgeführt.

Tabelle 2

| Temperatur | Druck | Reaktionszeit | Ausbeute IPAA/GC-% | Umsatz |
|---|---|---|---|---|
| 100°C | 50 bar | 5 h | 81,4 % | 100 % |

**Patentansprüche**

1. Einstufiges Verfahren zur Herstellung von 3-Aminomethyl-3,5,5-trimethylcyclohexanol, durch Hydrierung von Isophoronnitril (IPN) mit Wasserstoff an einem Katalysator, in An- oder Abwesenheit von Lösungsmittel, wobei der Katalysator die folgenden Eigenschaften aufweist:

I. Der Katalysator liegt in Form von katalytisch aktiven Metallschaumkörpern vor und weist eine Schüttdichte von nicht mehr als 0,8 kg/l auf,
und
II. Der Katalysator weist im aktivierten Zustand in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 70 bis 99,8 Gew.-% |
| Aluminium: | 0,02 bis 15 Gew.-% |

sowie eines oder mehrere Elemente ausgewählt aus der Gruppe bestehend aus Chrom, Nickel und Eisen: 0 bis 15 Gew.-%, und
III. die katalytisch aktiven Metallschaumkörper weisen eine durchschnittliche Teilchengröße von 1 bis maximal 70 mm auf,
und
IV. die katalytisch aktiven Metallschaumkörper zeigen eine poröse Schaumstruktur, wobei die makroskopischen Poren Größen im Bereich von 100 bis 5000 µm aufweisen.

2. Verfahren nach Anspruch 1, **dadurch gekennzeichnet, dass** der Katalysator eine Schüttdichte von 0,1 bis 0,7 kg/l, besonders bevorzugt von 0,2 bis 0,6 kg/l, aufweist.

3. Verfahren nach mindestens einem der vorherigen Ansprüche, **dadurch gekennzeichnet, dass** der Katalysator bevorzugt 70 bis 95 Gew.-%, besonders bevorzugt 80 bis 90 Gew. % Cobalt und 0,02 bis 15 Gew.-%, bevorzugt 4 bis 13 Gew.-%, ganz besonders bevorzugt 6 bis 12 Gew.-% Aluminium und weiterhin 0 bis 15 Gew.-%, bevorzugt 0,05 bis 8 Gew.-%, besonders bevorzugt 0,1 bis 3,5 Gew.-% und ganz besonders bevorzugt 0,2 bis 2,5 Gew.-% eines oder mehrere Elemente ausgewählt aus der Gruppe bestehend aus Chrom, Nickel und Eisen, enthält

4. Verfahren nach mindestens einem der vorherigen Ansprüche, **dadurch gekennzeichnet, dass** der Katalysator im aktivierten Zustand in seiner Gesamtheit die folgende Zusammensetzung aufweist, in Gewichtsprozent (Gew.-%), wobei sich die Anteile zu 100 Gew.-% addieren, bezogen auf die enthaltenden Metalle:

| | |
|---|---|
| Cobalt: | 80 bis 90 Gew.-% |
| Aluminium: | 6 bis 12 Gew.-% |

sowie eines oder mehrere Elemente ausgewählt aus der Gruppe bestehend aus Chrom, Nickel und Eisen: 0,05 bis 8 Gew.-%.

5. Verfahren nach mindestens einem der vorherigen Ansprüche, **dadurch gekennzeichnet, dass** der Katalysator im aktivierten Zustand in seiner Gesamtheit die folgende Zusammensetzung aufweist, in Gewichtsprozent (Gew.-%), wobei sich die Anteile zu 100 Gew.-% addieren, bezogen auf die enthaltenden Metalle:

| | |
|---|---|
| Cobalt: | 80 bis 90 Gew.-% |
| Aluminium: | 6 bis 12 Gew.-% |

sowie eines oder mehrere Elemente ausgewählt aus der Gruppe bestehend aus Chrom, Nickel und Eisen: 0,1 bis 3,5 Gew.-%.

6. Verfahren nach mindestens einem der vorherigen Ansprüche, **dadurch gekennzeichnet, dass** der Katalysator 0 bis 10 Gew.-% Dotiermetalle und/oder Promotoren zur Erhöhung der Aktivität, Selektivität und/oder Standzeit, enthält

7. Verfahren nach mindestens einem der vorherigen Ansprüche, **dadurch gekennzeichnet, dass** die katalytisch aktiven Metallschaumkörper eine Teilchengröße von 1 bis zu 50 mm, besonders bevorzugt von 1 bis zu 30 mm und ganz besonders bevorzugt von 1 bis nicht mehr als 10 mm, aufweisen.

8. Verfahren nach mindestens einem der vorherigen Ansprüche, **dadurch gekennzeichnet, dass** die katalytisch aktiven Metallschaumkörper des Katalysators makroskopischen Poren der Größen im Bereich von 100 bis 5000

μm, bevorzugt von 200 bis 2500 μm, besonders bevorzugt von 400 bis 1200 μm aufweisen.

**9.** Verfahren nach mindestens einem der vorherigen Ansprüche, **dadurch gekennzeichnet, dass** der Katalysator eine BET Oberfläche von 10 bis 120 $m^2/g$, besonders bevorzugt 70 bis 100 $m^2/g$, aufweist.

**10.** Verfahren nach mindestens einem der vorherigen Ansprüche, **dadurch gekennzeichnet, dass** die Hydrierung bei einer Temperatur von 20 bis 200°C und bei einem Druck von 0,3 bis 50 MPa durchgeführt wird.

**11.** Verfahren nach mindestens einem der vorherigen Ansprüche, **dadurch gekennzeichnet, dass** die Hydrierung einstufig in Batch-Autoklaven oder Festbettreaktoren erfolgt.

**12.** Verfahren nach mindestens einem der vorherigen Ansprüche, **dadurch gekennzeichnet, dass** der für die Hydrierung erforderliche Wasserstoff dem Reaktor entweder im Überschuss, bevorzugt mit bis zu 10000 Moläquivalenten, zugeführt wird, oder in einer solchen Menge, dass der durch Reaktion verbrauchte Wasserstoff sowie der Teil des Wasserstoffs, der eingelöst im Produktstrom den Reaktor verlässt, nachgeführt wird.

**13.** Verfahren nach mindestens einem der vorherigen Ansprüche, **dadurch gekennzeichnet, dass** Hydrierung kontinuierlich in Festbettreaktoren erfolgt.

**14.** Verfahren nach mindestens einem der vorherigen Ansprüche, **dadurch gekennzeichnet, dass** die Hydrierung kontinuierlich in Festbettreaktoren, die in Rieselbettfahrweise oder Sumpffahrweise betrieben werden, durchgeführt wird.

**15.** Verfahren nach mindestens einem der vorherigen Ansprüche, **dadurch gekennzeichnet, dass** das die Hydrierung verlassende Reaktionsgemisch einstufig oder mehrstufig aufgereinigt wird, und das 3-(Aminomethyl)-3,5,5-trimethylcyclohexanolerhalten wird.

Europäisches Patentamt
European Patent Office
Office européen des brevets

# EUROPÄISCHER RECHERCHENBERICHT

Nummer der Anmeldung
EP 18 15 6018

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | EP 3 050 870 A1 (EVONIK DEGUSSA GMBH [DE]) 3. August 2016 (2016-08-03)<br>* Absatz [0004] *<br>* Absätze [0012] - [0041] *<br>----- | 1-15 | INV.<br>C07C213/00<br>C07C215/42 |
| A,D | WO 2011/095576 A1 (HOFFMANN LA ROCHE [CH]; CHEN LI [CN]; LIN XIANFENG [CN]; QIU ZONGXING) 11. August 2011 (2011-08-11)<br>* Seite 57, Zeilen 2-13 *<br>----- | 1-15 | |

| RECHERCHIERTE SACHGEBIETE (IPC) |
|---|
| C07C |

Der vorliegende Recherchenbericht wurde für alle Patentansprüche erstellt

2

| Recherchenort | Abschlußdatum der Recherche | Prüfer |
|---|---|---|
| Den Haag | 4. Juni 2018 | Fitz, Wolfgang |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 18 15 6018

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-06-2018

| Im Recherchenbericht angeführtes Patentdokument | Datum der Veröffentlichung | Mitglied(er) der Patentfamilie | Datum der Veröffentlichung |
|---|---|---|---|
| EP 3050870 A1 | 03-08-2016 | CN 107428673 A | 01-12-2017 |
| | | EP 3050870 A1 | 03-08-2016 |
| | | JP 2018505175 A | 22-02-2018 |
| | | US 2017362163 A1 | 21-12-2017 |
| | | WO 2016120235 A1 | 04-08-2016 |
| WO 2011095576 A1 | 11-08-2011 | US 2011195979 A1 | 11-08-2011 |
| | | WO 2011094953 A1 | 11-08-2011 |
| | | WO 2011095576 A1 | 11-08-2011 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2011095576 A
- DE 1229078
- WO 2011094953 A
- EP 42119 A
- EP 659734 A
- EP 503246 A
- WO 2012076315 A
- EP 3050870 A


### In der Beschreibung aufgeführte Nicht-Patentliteratur


- **PANDEY et al.** *Indian Journal of Chemistry,* 2004, vol. 43B, 2705-2707
- Metallic Foams. Ullmann's Encyclopedia of Industrial Chemistry. 15. Juli 2012
- **PAUL SCHMIDT ; ROLF KÖRBER ; MATTHIAS COPPERS.** Sieben und Siebmaschinen: Grundlagen und Anwendung. Wiley-VCH Verlag, 2003
- *J. Am. Chem. Soc.,* 1938, vol. 60, 309-319
- The Guide 2000 of Technical Foams. 33-41